# EUROPÄISCHE PATENTANMELDUNG

(11) **EP 0 534 219 A1**
(43) Veröffentlichungstag der Anmeldung: **31.03.1993**
(21) Anmeldenummer: 92115272.4
(22) Anmeldetag: 07.09.1992
(51) Int. Cl.: C07D 285/08

(54) **3-(4-Bromphenyl)-5-methylsulfonyl-1,2,4-thiadiazol, ein Verfahren zur Herstellung, seine Verwendung und neue Zwischenprodukte**

(30) Priorität: 19.09.1991 DE 4131138
(71) Anmelder: BAYER AG, 51368 Leverkusen (DE)
(72) Erfinder: Diehr, Hans-Joachim, Dr., W-5600 Wuppertal 11 (DE); Dehne, Heinz-Wilhelm, Dr., W-4019 Monheim (DE)

(57) **Zusammenfassung**

3-(4-Bromphenyl)-5-methylsulfonyl-1,2,4-thiadiazol der Formel (I) 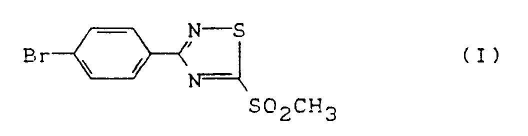
seine Herstellung und vorzugsweise Verwendung als Fungizid.

Die neue Verbindung der Formel (I) kann aus der entsprechenden Methylmercaptoverbindung mit geeigneten Oxidationsmitteln hergestellt werden. Die Methylmercaptoverbindung ist ebenfalls neu und kann durch geeignete Methylierungsmittel aus der Mercaptoverbindung hergestellt werden, die aus dem entsprechenden Amidin durch Ringschluß hergestellt wird.

## Beschreibung

Die vorliegende Erfindung betrifft das neue 3-(4-Bromphenyl)-5-methylsulfonyl-1,2,4-thiadiazol, ein Verfahren zu seiner Herstellung, seine Verwendung z.B. in Schädlingsbekämpfungsmitteln, vor allem als Fungizid und neue Ausgangs- bzw. Zwischenprodukte.

Es ist bereits bekannt, daß bestimmte substituierte 1,2,4-Thiadiazole, wie beispielsweise 5-Methylsulfonyl-3-phenyl-1,2,4-thiadiazol nematizide und fungizide Eigenschaften besitzen (vgl. DE-OS 2 142 913).

Außerdem ist die bakterizide und fungizide Wirkung von 3-(4-Chlorphenyl)-5-methylsulfonyl-1,2,4-thiadiazol bekannt (vgl. DE-OS 2 242 187).

Die Wirkung dieser Verbindungen ist jedoch insbesondere bei niedrigen Aufwandmengen und Konzentrationen nicht immer in allen Anwendungsbereichen völlig zufriedenstellend.

Überraschenderweise wurde gefunden, daß das neue 3-(4-Bromphenyl)-5-methylsulfonyl-1,2,4-thiadiazol der Formel (I) 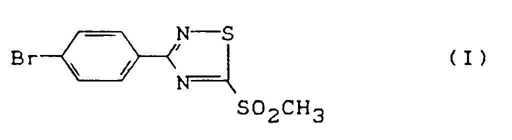
eine sehr gute fungizide Wirksamkeit besitzt und somit eine Bereicherung der Technik darstellt.

Weiterhin wurde gefunden, daß man das neue 3-(4-Bromphenyl)-5-methylsulfonyl-1,2,4-thiadiazol der Formel (I) 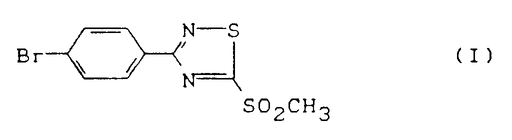
erhält, wenn man
3-(4-Bromphenyl)-5-methylmercapto-1,2,4-thiadiazol der Formel (II) 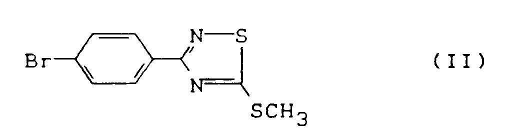
mit einem Oxidationsmittel, gegebenenfalls in Gegenwart eines Verdünnungsmittels und gegebenenfalls in Gegenwart eines Katalysators, umsetzt.

Verwendet man als Ausgangsstoffe beispielsweise 3-(4-Bromphenyl)-5-methylmercapto-1,2,4-thiadiazol und zur Oxidation Wasserstoffperoxid, so läßt sich der Reaktionsablauf des erfindungsgemäßen Verfahrens durch das folgende Formelschema darstellen: 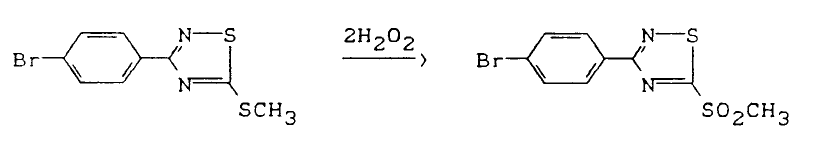
Das zur Durchführung des erfindungsgemäßen Verfahrens benötigte 3-(4-Bromphenyl)-5-methylmercapto-1,2,4-thiadiazol der Formel (II) ist neu und ebenfalls Gegenstand der Erfindung und kann nach allgemein bekannten Verfahren analog erhalten werden, wenn man beispielsweise 3-(4-Bromphenyl)-5-mercapto-1,2,4-thiadiazol der Formel (III) 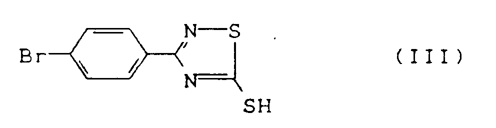
mit Methylierungsmitteln, wie beispielsweise Methyliodid in Gegenwart einer Base, wie beispielsweise Kaliumcarbonat und in Gegenwart eines Verdünnungsmittels, wie beispielsweise Aceton, Methylethylketon oder Acetonitril bei Temperaturen zwischen 20° bis 100°C methyliert (vgl. das Herstellungsbeispiel).

Die Verbindung der Formel (III) ist ebenfalls neu und kann nach allgemein bekannten Methoden in analoger Weise hergestellt werden (vgl. z.B. DE-OS 16 70 068, entsprechend US 3 478 045; Adv. Het. Chem. 5, 133, 1965; US 4 758 578 und Herstellungsbeispiel).

Die Oxidationsmittel für das erfindungsgemäße Verfahren sind an sich bekannt (vergl. Reaktionen der organischen Synthese, G. Thieme Verlag, Stuttgart (1978), S. 472).

Beispielsweise seien Wasserstoffperoxid, Perameisensäure, Peressigsäure, Perbenzoesäure, m-Chlorperbenzoesäure und/oder Natriummetaperiodat und wässrige Lösungen von Halogen, insbesondere Chlor oder Brom genannt (vgl. S. Oae, Organic Chemistry of Sulfur, Plenum Press, New York, 1977, S.529ff).
Bevorzugte Oxidationsmittel sind Wasserstoffperoxid und/oder Persäuren.

Die Menge an Oxidationsmittel beträgt im allgemeinen etwa 1 bis 4 Äquivalente, bezogen auf 3-(4-Bromphenyl)-5-methylmercapto-1,2,4-thiadiazol der Formel (II).

Als Verdünnungsmittel für die Oxidationsreaktion kommen alle inerten organischen Lösungsmittel in Frage, die sich unter den Oxidationsbedingungen nicht verändern.

Hierzu gehören vorzugsweise chlorierte Kohlenwasserstoffe, wie Methylenchlorid, Chloroform, Tetrachlorkohlenstoff, 1,2-Dichlorethan, 1,2,2-Trichlorethan und Chlorbenzol; Alkohole, vorzugsweise Methanol, Ethanol oder Isopropanol; niedere Fettsäuren, bevorzugt Ameisensäure, Essigsäure oder Propionsäure. Das erfindungsgemäße Verfahren kann auch in Mischung der Lösungsmittel mit Wasser oder in Gemischen von mehreren Lösungsmitteln durchgeführt werden.

Als Katalysator bei der Oxidation können die üblicherweise hierfür verwendeten Salze von Metallen der IV., V. und VI. Nebengruppe des Periodensystems der Elemente verwendet werden, insbesondere seien Vanadiumpentoxid, Natriummetavanadat, Natriummolybdat und/oder Natriumwolframat genannt.

Die günstigste Menge an Oxidationsmittel und Katalysator bei der Oxidation kann leicht durch Vorversuche ermittelt werden. Üblicherweise wird die oben angegebene Menge des Oxidationsmittels mit etwa 0,01 bis 0,1, bevorzugt 0,015 bis 0,09, Mol Katalysator je Mol 3-(4-Bromphenyl)-5-methylmercapto-1,2,4-thiadiazol der Formel (II) eingesetzt. Die Verdünnungsmittelmenge ist nicht kritisch und kann ebenfalls leicht durch Vorversuche ermittelt werden.

Die Oxidation wird im allgemeinen im Temperaturbereich von ca. -60 °C bis +120 °C, vorzugsweise im Temperaturbereich von -60 °C bis +80 °C durchgeführt.

Die Oxidation kann bei Normaldruck, aber auch bei einem Unter- oder Überdruck (beispielsweise im Druckbereich von 0,5 bis 1,5 bar) durchgeführt werden. Bevorzugt führt man die Oxidation bei Normaldruck aus.

Bei der Herstellung von 3-(4-Bromphenyl)-5-methylsulfonyl-1,2,4-thiadiazol der Formel (I) nach dem erfindungsgemäßen Verfahren setzt man im allgemeinen jeweils die äquivalente Menge an Oxidationsmittel ein, d. h. man setzt pro Mol 3-(4-Bromphenyl)-5-methylmercapto-1,2,4-thiadiazol der Formel (II) vier Oxidationsäquivalente, gegebenenfalls auch einen größeren Überschuß, ein.

Die Isolierung des 3-(4-Bromphenyl)-5-methylsulfonyl-1,2,4-thiadiazol der Formel (I) erfolgt in allgemein üblicher Art und Weise, beispielsweise durch Versetzen des Reaktionsgemisches mit Wasser, Extraktion mit einem organischen Lösungsmittel, Einengen und gegebenenfalls Umkristallisieren.

Der erfindungsgemäße Wirkstoff weist eine starke biologische Wirkung auf und kann zur Bekämpfung von unerwünschten Schädlingen praktisch eingesetzt werden. Die Wirkstoffe sind z.B.für den Gebrauch als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise
Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit des Wirkstoffes in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel kann der erfindungsgemäße Wirkstoff mit besonders gutem Erfolg protektiv zur Bekämpfung von Phythophthora-Arten an Tomaten, Plasmopara-Arten an Reben und Botrytis-Arten an Bohnen eingesetzt werden.

Außerdem besitzt der erfindungsgemäße Wirkstoff eine gute fungizide Wirkung gegen Pyricularia oryzae an Reis und gegen Septoria nodorum. Außerdem zeigt er auch eine breite Wirkung im in-vitro-Test.

Der Wirkstoff kann in Abhängigkeit von seinen jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut sowie ULV-Kalt- und Warmnebel-Formulierungen.

Diese Formulierungen werden in bekannter Weise hergestellt, z.B. durch Vermischen des Wirkstoffs mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Der Wirkstoff kann als solcher, in Form seiner Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, Vorzugsweise von 0,0001 bis 0,02 % am Wirkungsort erforderlich.

Die Herstellung und die Verwendung des erfindungsgemäßen Wirkstoffes geht aus den folgenden Beispielen hervor.

### Herstellungsbeispiel

### Beispiel 1:

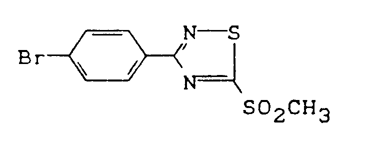

Zu einer Lösung von 5,5 g (0,019 Mol) 3-(4-Bromphenyl)-5-methylmercapto-1,2,4-thiadiazol in 50 ml Eisessig und einer Spatelspitze Natriumwolframat werden bei 50-60°C 17 g (ca.15 ml, 0,17 Mol) einer 35%igen wäßrigen Wasserstoffperoxid-Lösung getropft. Die Reaktionsmischung wird 15 Minuten bei dieser Temperatur nachgerührt, abgekühlt und mit Wasser verdünnt. Der kristalline Feststoff wird abgesaugt, mit kaltem Wasser gewaschen und getrocknet.

Es werden 5,9 g (97,4% der Theorie) kristallines 3-(4-Bromphenyl)-5-methylsulfonyl-1,2,4-thiadiazol vom Schmelzpunkt 131-32°C erhalten.

### Herstellung der Ausgangsverbindungen

a) 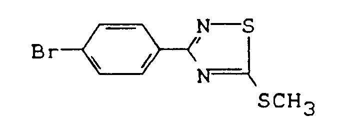 Zu 6,8 g (0,025 Mol) 3-(4-Bromphenyl)-5-mercapto-1,2,4-thiadiazol und 5 g (0,036 Mol) Kaliumcarbonat in 80 ml Aceton werden bei Raumtemperatur 3,6 g (0,025 Mol) Methyliodid tropfenweise hinzugesetzt und das Gemisch 15 Minuten bei 30°C nachgerührt. Nach vollständigem Umsatz wird das anorganische Salz abgesaugt und das Filtrat im Vakuum eingeengt. Der erhaltene Rückstand wird mit Methyl-tert.-butylether gewaschen und getrocknet.
   Man erhält 5,5 g (77% der Theorie) 3-(4-Bromphenyl)-5-methylmercapto-1,2,4-thiadiazol vom Schmelzpunkt: 101°C.
b) 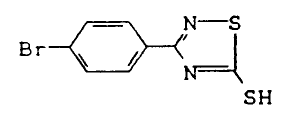 Ein Gemisch aus 29,6 g (0,125 Mol) 4-Brombenzamidin-hydrochlorid, 81 g (0,375 Mol) einer methanolischen 25,1%igen Natriumethylat-Lösung, 24 g (0,315 Mol) Schwefelkohlenstoff, 4,2 g (0,13 g-Atom) Schwefel und 100 ml Methanol wird 8 Stunden unter Schwefelwasserstoffentwicklung unter Rückfluß erhitzt. Nach Ausblasen des restlichen Schwefelwasserstoffs durch Stickstoff und Filtration der Lösung wird das Lösungsmittel im Vakuum abdestilliert. Der feste Rückstand wird in Wasser gelöst und die filtrierte Lösung mit Eisessig angesäuert. Der Feststoff wird abgesaugt, nacheinander mit Wasser und Diisopropylether gewaschen und getrocknet.

Es werden 23,9 g (70% der Theorie) 3-(4-Bromphenyl)-5-mercapto-1,2,4-thiadiazol als bräunliches Kristallmehl mit einem Schmelzpunkt von 165°C erhalten.

### Anwendungsbeispiele

In den folgenden Anwendungsbeispielen wird die nachfolgend aufgeführte Verbindung als Vergleichssubstanz eingesetzt: 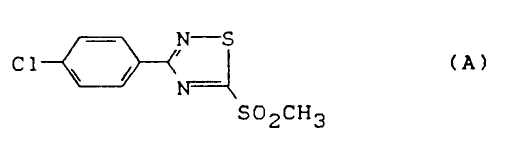
3-(4-Chlorphenyl)-5-methylsulfonyl-1,2,4-thiadiazol (bekannt aus DE-OS 2242187).

### Beispiel A

### Phytophthora-Test (Tomate)/protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigt in diesem Test die Verbindung 1.

### Beispiel B

### Plasmopara-Test (Reben) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

6 Tage nach der Inokulation erfolgt die Auswertung.

Eine deutliche Überlegenheit in der Wirksamkeit gegenüber dem Stand der Technik zeigt in diesem Test die Verbindung 1.

### Beispiel C

### Botrytis-Test (Buschbohne)/protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe, Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigt in diesem Test die Verbindung 1.

## Patentansprüche

1. 3-(4-Bromphenyl)-5-methylsulfonyl-1,2,4-thiadiazol der Formel (I)

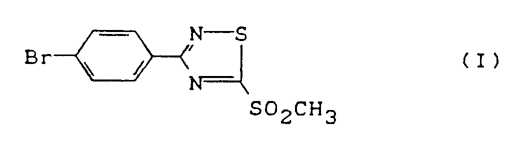

2. Verfahren zur Herstellung von 3-(4-Bromphenyl)-5-methylsulfonyl-1,2,4-thiadiazol der Formel (I) 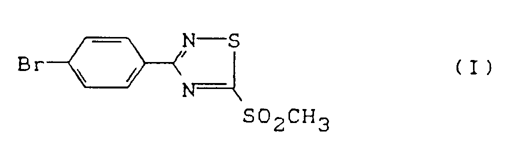 dadurch gekennzeichnet, daß man 3-(4-Bromphenyl)-5-methylmercapto -1,2,4-thiadiazol der Formel (II) 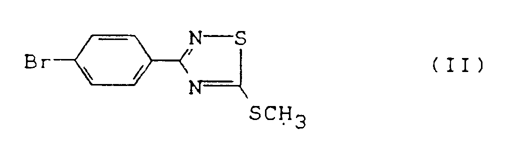 mit einem Oxidationsmittel, gegebenenfalls in Gegenwart eines Verdünnungsmittels und gegebenenfalls in Gegenwart eines Katalysators, umsetzt.

3. 3-(4-Bromphenyl)-5-methylmercapto-1,2,4-thiadiazol der Formel (II)

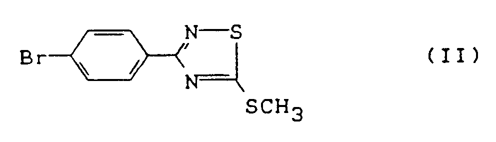

4. Verfahren zur Herstellung von 3-(4-Bromphenyl)-5-methylmercapto-1,2,4-thiadiazol der Formel (II) 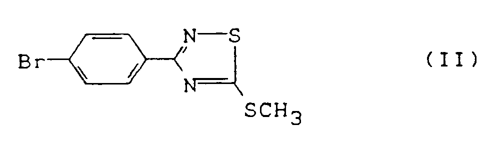 dadurch gekennzeichnet, daß man 3-(4-Bromphenyl)-5-mercapto-1,2,4-thiadiazol der Formel (III) 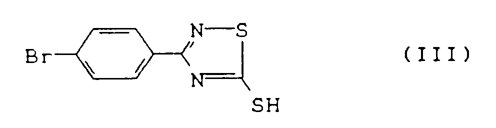 mit Methylierungsmitteln in Gegenwart einer Base und in Gegenwart eines Verdünnungsmittels umsetzt.

5. 3-(4-(Bromphenyl)-5-mercapto-1,2,4-thiadiazol der Formel (III)

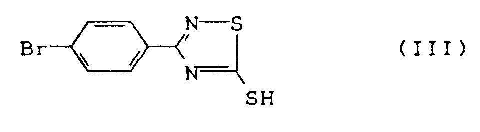

6. Verfahren zur Herstellung von 3-(4-(Bromphenyl)-5-mercapto-1,2,4-thiadiazol der Formel (III) 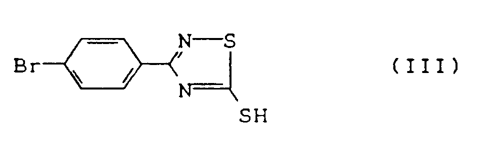 dadurch gekennzeichnet, daß man 4-Brombenzamidinhydrochlorid der Formel 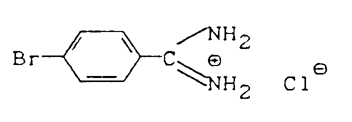 mit Schwefelkohlenstoff und Schwefel in Alkoholat-Lösung umsetzt.

7. Schädlingsbekämpfungsmittel, gekennzeichnet durch einen Gehalt an 3-(4-Bromphenyl)-5-methylsulfonyl-1,2,4-thiadiazol der Formel (I) nach den Ansprüchen 1 und 2.

8. Verwendung von 3-(4-Bromphenyl)-5-methylsulfonyl-1,2,4-thiadiazol der Formel (I) nach den Ansprüchen 1 und 2 zur Bekämpfung von Schädlingen.

9. Verfahren zur Bekämpfung von Schädlingen, dadurch gekennzeichnet, daß man 3-(4-Bromphenyl)-5-methylsulfonyl-1,2,4-thiadiazol der Formel (I) nach den Ansprüchen 1 und 2 auf Schädlinge und/oder ihren Lebensraum einwirken läßt.

10. Verfahren zur Herstellung von Schädlingsbekämpfungsmittel, dadurch gekennzeichnet, daß man 3-(4-Bromphenyl)-5-methylsulfonyl-1,2,4-thiadiazol der Formel (I) nach den Ansprüchen 1 und 2 mit Streckmitteln und/oder oberflächenaktiven Mitteln vermischt.

11. Verwendung der Verbindungen der Formeln (II) und (III) gemäß den Ansprüchen 3 und 5 als Zwischenprodukte zur Herstellung hochaktiver Verbindungen.
